# EUROPEAN PATENT SPECIFICATION

(11) **EP 3 966 575 B1**
(45) Date of publication and mention of the grant of the patent: **23.08.2023**
(21) Application number: 20722365.2
(22) Date of filing: 06.05.2020
(51) Int. Cl.: G01N 33/68, G01N 33/84

(54) **CYSTIC FIBROSIS URINE TEST**
URINTEST AUF ZYSTISCHE FIBROSE
TEST D'URINE POUR LA MUCOVISCIDOSE

(30) Priority: 08.05.2019 EP 19173359
(43) Date of publication of application: 16.03.2022
(73) Proprietor: Aarhus Universitet, 8000 Aarhus C (DK); University of Regensburg, 93053 Regensburg (DE)
(72) Inventor: LEIPZIGER, Jens, Georg, 8000 Aarhus C (DK); KUNZELMANN, Karl, 93051 Regensburg (DE); BERG, Peder, 8000 Aarhus C (DK)
(74) Representative: Plougmann Vingtoft a/s
(86) International application number: PCT/EP2020/062582
(87) International publication number: WO 2020/225302

(56) References cited:
- US-A1- 2014 073 632
- US-A1- 2018 153 826
- MUJAN VARASTEH KIA ET AL: "Downregulation of the Cl-/HCO3- Exchanger Pendrin in Kidneys of Mice with Cystic Fibrosis: Role in the Pathogenesis of Metabolic Alkalosis", CELLULAR PHYSIOLOGY AND BIOCHEMISTRY., vol. 45, no. 4, 1 January 2018 (2018-01-01), pages 1551-1565, XP055639883, CH ISSN: 1015-8987, DOI: 10.1159/000487691
- KUNZELMANN KARL ET AL: "Bicarbonate in cystic fibrosis", JOURNAL OF CYSTIC FIBROSIS, vol. 16, no. 6, 18 July 2017 (2017-07-18), pages 653-662, XP085262737, ISSN: 1569-1993, DOI: 10.1016/J.JCF.2017.06.005 & D Bretscher ET AL: "Response of renal handling of sodium (Na) and bicarbonate (HCO 3 - ) to secretin (S) in normals and patients with cystic fibrosis (CF). | Pediatric Research", , 1 November 1974 (1974-11-01), XP055639895, Retrieved from the Internet: URL:https://www.nature.com/articles/pr1974 1007 [retrieved on 2019-11-07]
- EK SCHNEIDER ET AL: "Can Cystic Fibrosis Patients Finally Catch a Breath With Orkambi?", CLINICAL PHARMACOLOGY AND THERAPEUTICS, vol. 101, no. 1, 23 November 2016 (2016-11-23), pages 130-141, XP055639902, US ISSN: 0009-9236, DOI: 10.1002/cpt.548
- A. L. VITERI ET AL: "Renal response to secretin", JOURNAL OF APPLIED PHYSIOLOGY, vol. 38, no. 4, 1 April 1975 (1975-04-01), pages 661-664, XP055639405, US ISSN: 8750-7587, DOI: 10.1152/jappl.1975.38.4.661

## Description

### Technical field of the invention

The present invention relates to a method for determining the effectiveness of a compound in a subject for treatment of cystic fibrosis.

### Background of the invention

Cystic Fibrosis (CF) is the most common lethal genetic disease in Caucasians with a frequency of ~1 in 2000 newborns (O'Sullivan et al. 2009) caused by loss of function of the Cystic Fibrosis Transmembrane Regulator gene (CFTR).

CFTR is a cAMP-activated Cl-channel localized in the apical membrane of many glandular tissues (Jentsch et al. 2002). When defective, the balance of chloride and fluids is disrupted. Consequently, the cells produce mucus that is sticky and thicker than normal. This change in the mucus may lead to lung infections resulting potentially in respiratory failure in the lungs, poor digestion and infertility.

Many parts of the body are affected by CF, such as the lungs, liver, pancreas, urinary tract, reproductive organs and sweat glands. Back in the 1950s the sweat test was developed as a clinically relevant test for diagnosing CF, which otherwise can be difficult to clearly diagnose efficiently due to the many different symptoms observed in the patients (Meeting report: 2002). It has been observed that cystic fibrosis mice display an impaired ability to excrete bicarbonate as judged by reduction in urine pH and the generation of metabolic alkalosis (Mujan Varasteh Kia et al, Cellular Physiology and Biochemistry; 45(4), 1551-1565, 2018).

The discovery of the CFTR gene and its linkage with CF was thus, thought to be able to provide a sensitive and specific test for CF (Meeting report: 2002). However, a vast amount of different mutations have been found to cause CF. The mutations may be grouped into five different classes i.e. protein production mutations (class 1), protein processing mutations (class 2), gating mutations (class 3), conduction mutations (class 4) and insufficient protein mutations (class 5). Class 1 relates to mutations, where no functional CFTR is produced. Class 2 relates to mutations where a CFTR protein is synthetized but does not move to the cell surface as it is not correctly folded. Class 3 relates to a CFTR protein, which is integrated in the cell membrane but does not open the channel gate properly. Class IV relates to mutations where a CFTR protein is integrated in the cell membrane but the function of the channel is faulty. Class V relates to mutations, where a normal CFTR protein is created but in insufficient amounts.

It has been observed that specific mutations may be associated with specific disease complications but a clear genotype/phenotype correlation is not established. For the same reason some patients develop the symptoms of cystic fibrosis in the early years of life while others do not develop the symptoms until late childhood or adolescence even though they carry the same CFTR genotype.

Also, it is not always possible to organize all of the mutations in the different classes and neither is it certain that the mutations within one class will respond equally to the same treatment. Thus, a gene test did not turn out to be as informative as hoped for. Accordingly, the sweat test is still used for clinically following and testing the patients, the progress of their disease and the influence of drugs for CF.

The sweat test, however, is relative cumbersome to perform and demands specific equipment, why the test is performed in specialised centers alone. Furthermore, the false-positive and false-negative results are occasionally observed for the sweat test (Meeting report: 2002). Hence, an improved and simple test for measuring the influence of drugs and to classify groups of CF patients would be advantageous.

### Summary of the invention

The scope of the invention is defined by the appended claims. Embodiments and descriptions no longer falling under the scope of the appended claims are considered merely as examples suitable for understanding the invention.

Thus, an object of the present invention relates to the development of a simple test for measuring the effectiveness of a drug in the individual patients.

Thus the invention relates to a method of determining the effectiveness of a compound in a subject for treatment of cystic fibrosis comprising the steps of
a. determining a first excretion level of bicarbonate in a first urine sampling obtained from a subject having cystic fibrosis; where said first urine sampling has been obtained prior to said subject has been treated with a compound for treatment of cystic fibrosis;
b. determining a second excretion level of bicarbonate in a second urine sampling from said subject; where said second urine sampling is has been obtained after said subject has been treated with said compound for a predetermined time period;
c. determining the effectiveness of the compound against cystic fibrosis by comparing said first excretion level of bicarbonate to said second excretion level of bicarbonate;
wherein said compound is considered as effective if said second excretion level of bicarbonate is above said first excretion level of bicarbonate and said compound is considered ineffective if said second excretion level of bicarbonate is below or at the same excretion level as said first excretion level of bicarbonate; and wherein bicarbonate and/or secretin has been administered to said subject prior to having obtained said first urine sampling and prior to having obtained said second urine sampling; and wherein bicarbonate has been administered to said subject between 0-180 min prior to having obtained a first sample of said first urine sampling and prior to having obtained a first sample of said second urine sampling.

The disclosure further describes a method for sub-categorizing cystic fibrosis
a. determining a first excretion level of bicarbonate in a first urine sampling from a subject having one or more cystic fibrosis mutations, wherein bicarbonate and/or secretin has been administered to said subject prior to obtaining said first urine sampling;
b. determining whether the first excretion level of bicarbonate belongs to sub-category x1, x2 or x3; wherein x1, x2 and x3 are ranges defining different sub-categories of cystic fibrosis; and
wherein x1 is defined as the excretion level of bicarbonate being absent or nearly absent; x2 is defined as the excretion level of bicarbonate being significantly reduced and x3 is defined as the excretion level of bicarbonate being normal or almost normal.

Yet another aspect of the present invention is to provide a use of the excretion level of bicarbonate in the urine to determine the effectiveness of a compound against cystic fibrosis.

The disclosure further describes use of a composition comprising bicarbonate for diagnosing cystic fibrosis, wherein the composition is an acute challenge inducing a gastric load.

### Brief description of the figures

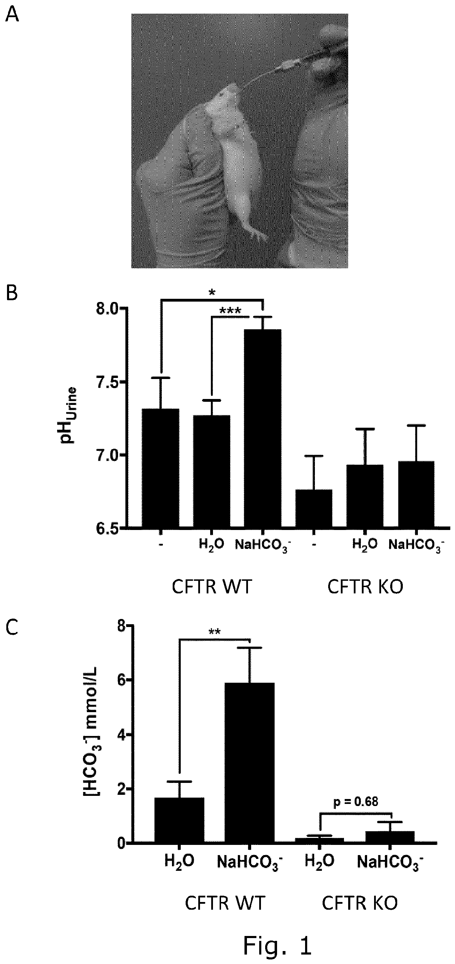

Figure 1 shows that a gastric load administered to mice **(A)** of bicarbonate. This causes an acute increase of urine pH and increases in [HCO₃-]ᵤ in CFTR wild type mice after 3 hrs, while in CFTR knock out mice this effect is absent **(B and C);**

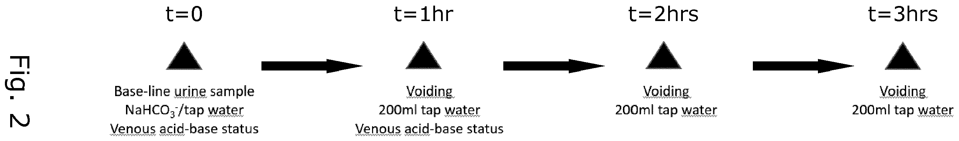

Figure 2 shows the test protocol as used in the human studies in schematic form;

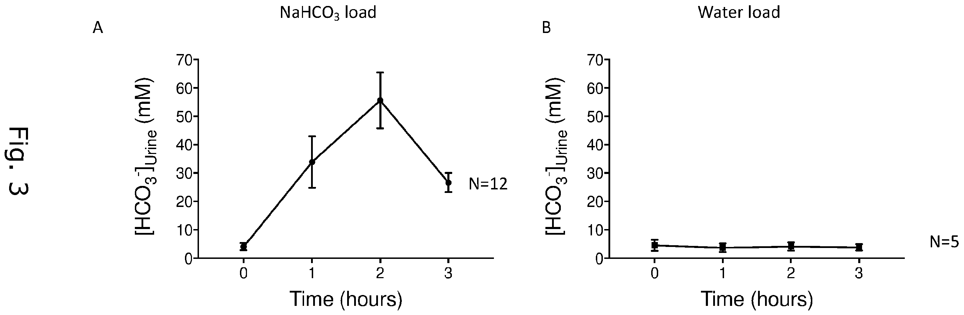

Figure 3 shows that an oral load of bicarbonate (79mg/Kg body weight (BW) NaHCO₃) rapidly increase [HCO₃⁻]ᵤ concentration in humans (12 healthy adults) **(A),** in controls receiving water **(B)** the [HCO₃-]ᵤ concentration remains unchanged.

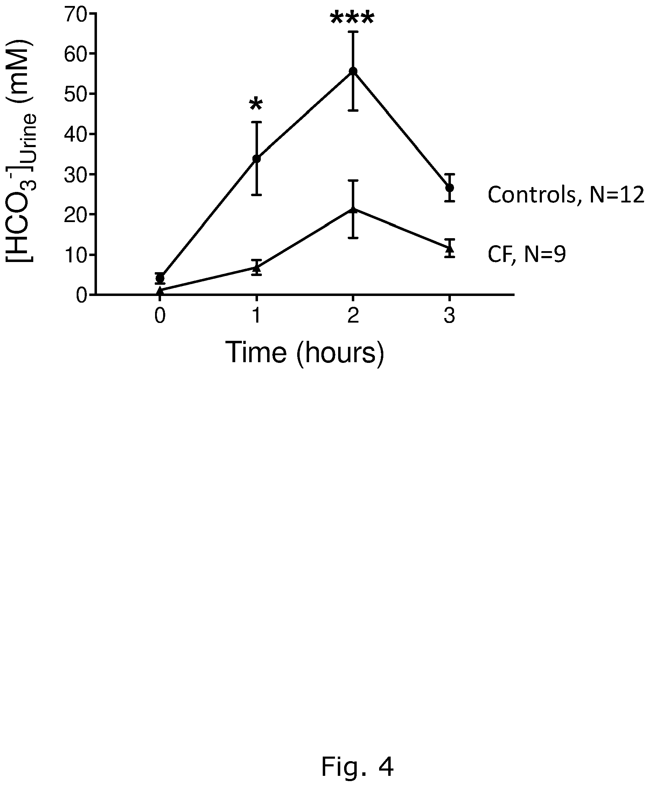

Figure 4 shows that urine HCO₃⁻ concentration increases after intake of 79 mg/Kg body weight (BW) NaHCO₃ in adult controls; in CF patients this increase is reduced.

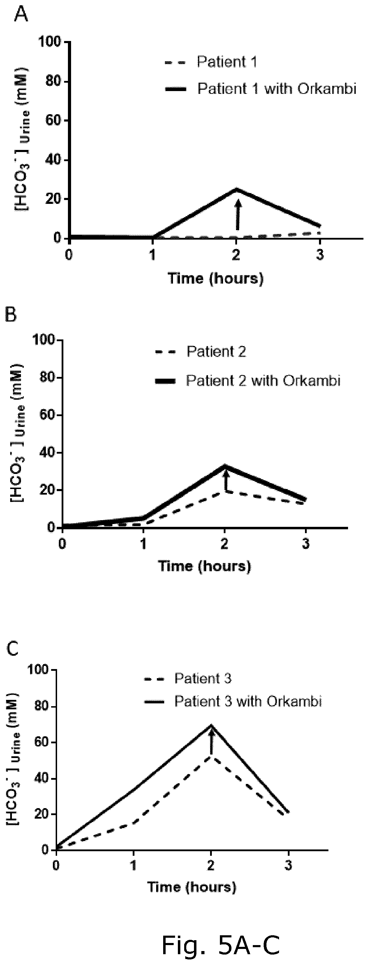

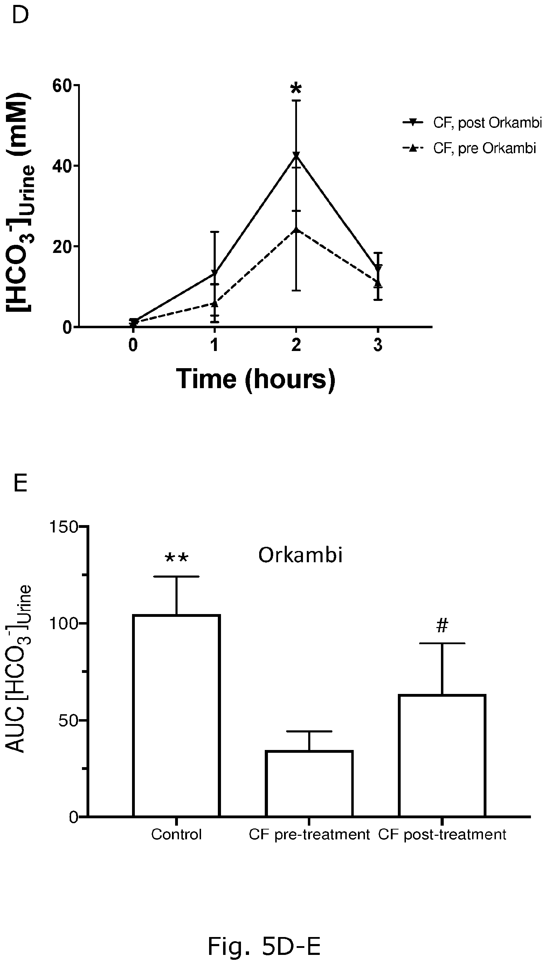

Figure 5 shows the CF urine test applied to 3 CF patients before and after 4 weeks treatment with Orkambi^{®}. **(A-C)** show the results of each individual CF patient. In **(D)** the mean of the individual results is shown, indicating significant treatment (*, p<0.05) success though a difference response pattern is observed for each CF patient. **(E)** demonstrates a mean of the results obtained in 5A-C calculated as the AUC₀₋₃ of excreted bicarbonate. ** denotes a significant (p<0.05) difference between control and CF pre-treatment. # denotes a significant (p<0.05) difference between CF pre-treatment and CF post-treatment.

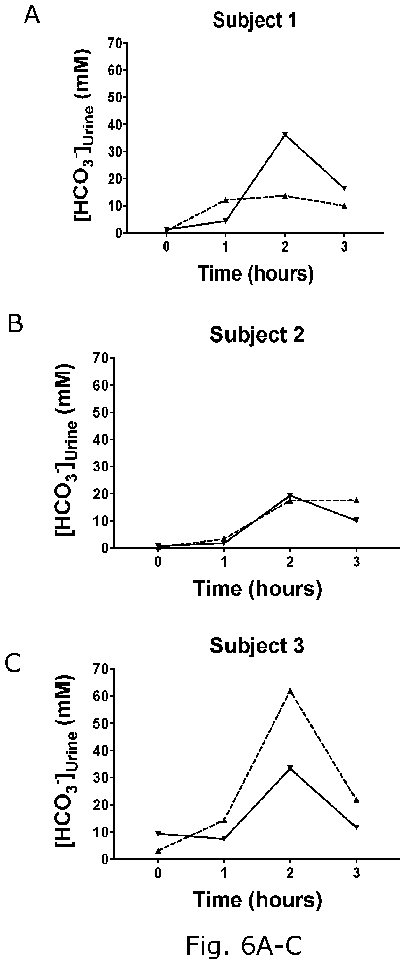

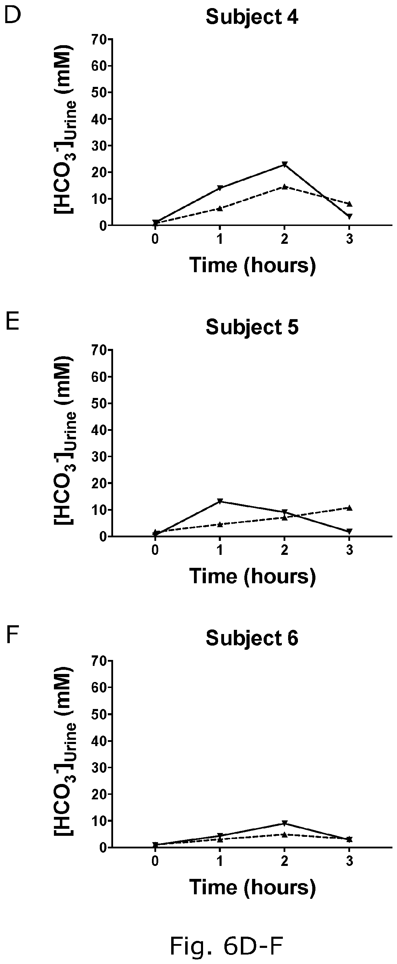

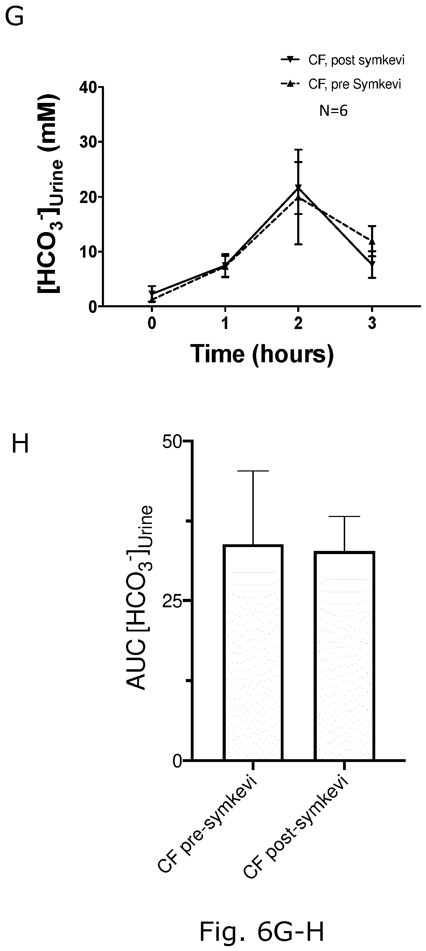

Figure 6 shows the CF urine test applied to 6 CF patients before and after 4 weeks treatment with Symkevi^{®}. **(A-F)** show the results of each individual CF patient. In **(G)** the mean of the individual results is shown. **(H)** demonstrates a mean of the results obtained in 6A-F calculated as the AUC₀₋₃ of excreted bicarbonate.

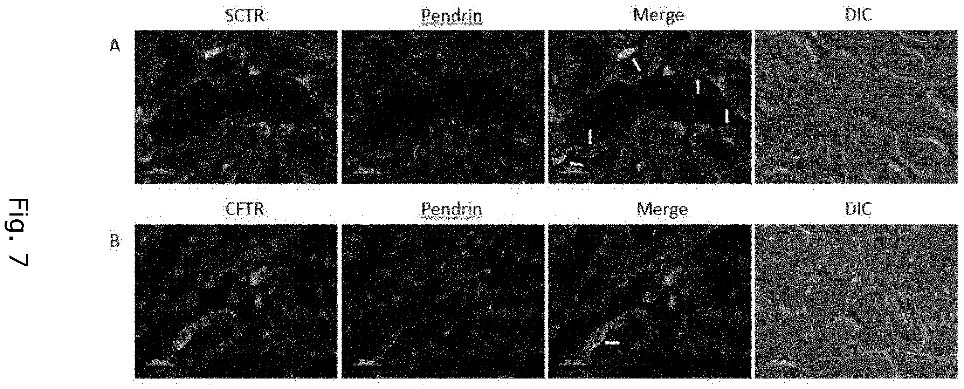

Figure 7 shows co-expression of secretin-receptor (SCTR) and CFTR in pendrin expressing beta intercalated cells. **(A)** SCTR is expressed on the basolateral membrane of tubular epithelial cells (green). Pendrin is expressed on the apical membrane of beta intercalated cells (red). Merged picture shows co-expression of SCTR and pendrin (arrows). **(B)** Tubular epithelial cells express CFTR in the apical and basolateral membrane of tubular epithelial cells (green). Merged picture shows co-expression of CFTR and pendrin (arrows). Bar 20 µm, differential interference contrast (DIC).

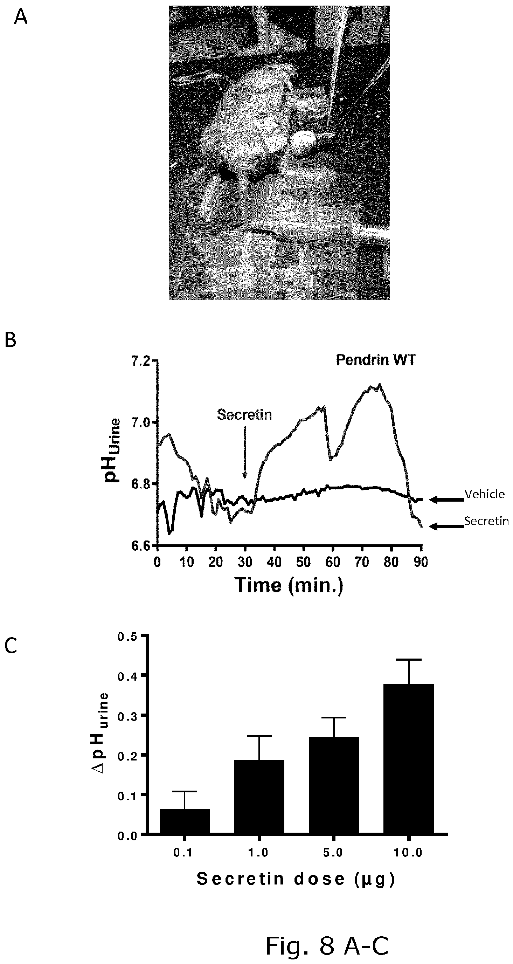

Figure 8 shows induction of an acute urinary alkalisation by secretin in wildtype (WT) mice. **(A)** shows the monitoring set-up in the mice. **(B)** shows the pH trace of urine measurement in pendrin WT mice with secretin or vehicle injection at the time of the vertical arrow (at 30 min). **(C)** shows the concentration dependent influence of secretin on the maximal difference in pH between the curves with and without secretin injection.

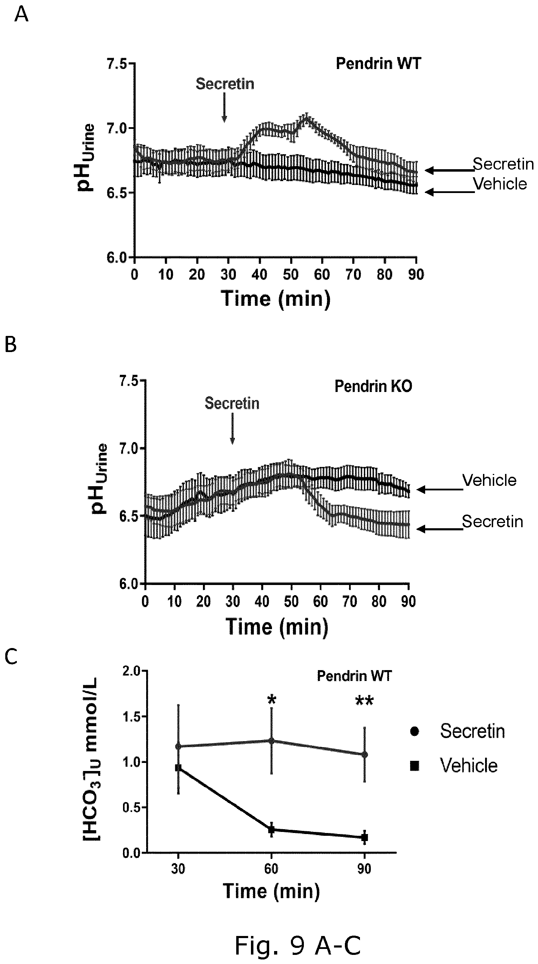

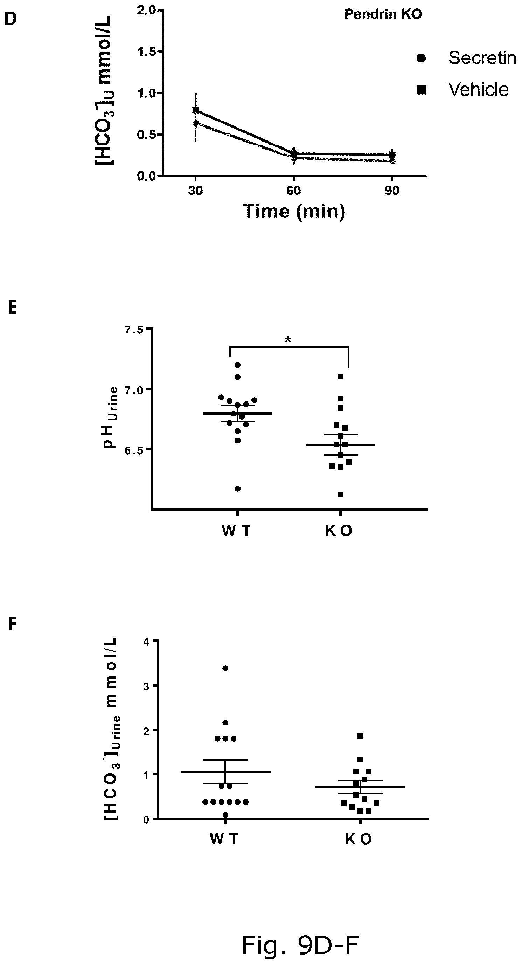

Figure 9 shows that secretin-induced urine alkalisation is absent in pendrin KO mice. **(A)** shows urine pH measured in Pendrin WT with secretin or vehicle injection at the time of the vertical arrow (at 30 min). **(B)** shows urine pH measured in Pendrin KO with secretin or vehicle injection at the time of the vertical arrow (at 30 min). **(C)** shows amount of bicarbonate measured in Pendrin WT with secretin or vehicle injection. **(D)** shows amount of bicarbonate measured in Pendrin KO with secretin or vehicle injection. **(E)** shows baseline measurements of the urine pH in WT and KO pendrin mice. **(F)** shows baseline measurements of the amount of bicarbonate in the urine measured in WT and KO pendrin mice.

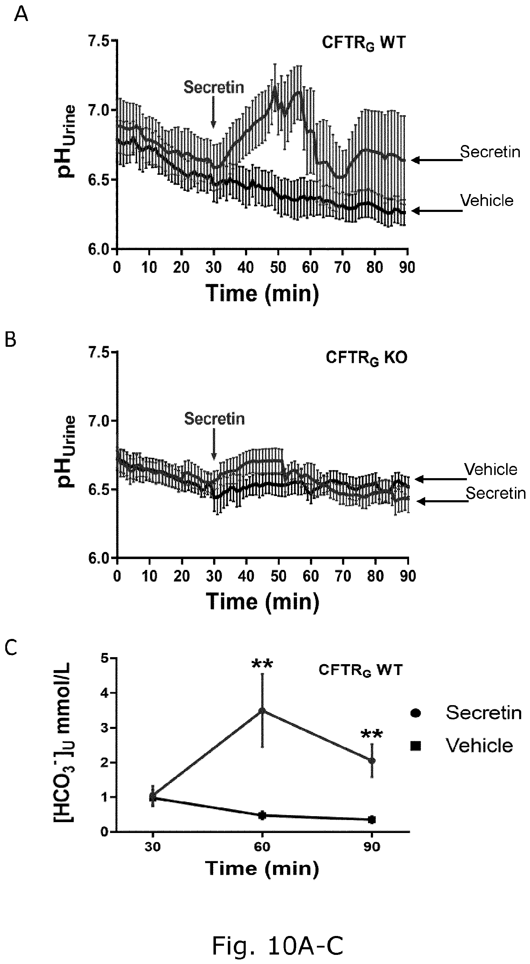

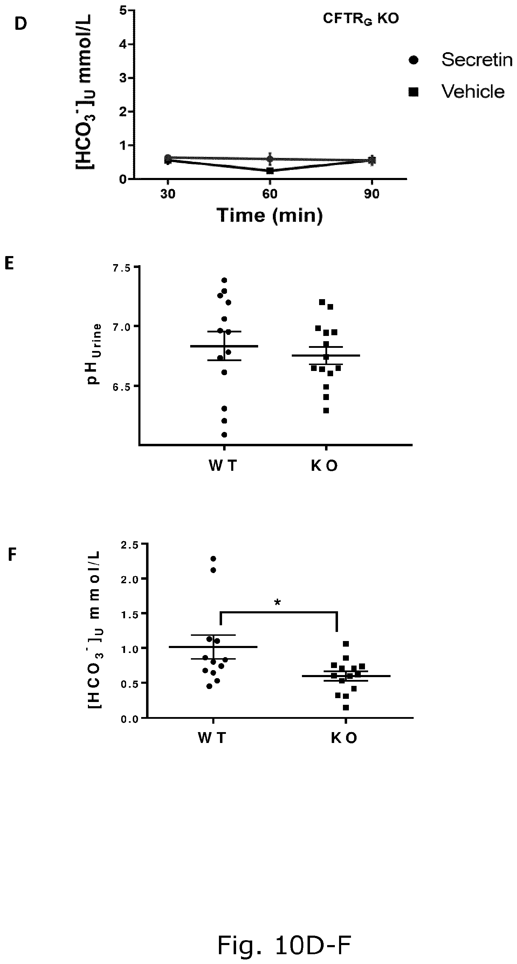

Figure 10 shows that secretin-induced urine alkalisation is absent in CFTR KO mice. **(A)** shows urine pH measured in CFTR WT with secretin or vehicle injection at the time of the vertical arrow (at 30 min). **(B)** shows urine pH measured in CFTR KO with secretin or vehicle injection at the time of the vertical arrow (at 30 min). **(C)** shows amount of bicarbonate measured in CFTR WT with secretin or vehicle injection. **(D)** shows amount of bicarbonate measured in CFTR KO with secretin or vehicle injection. **(E)** shows baseline measurements of the urinal pH in WT and KO CFTR mice. **(F)** shows baseline measurements of the amount of bicarbonate in the urine measured in WT and KO CFTR mice.

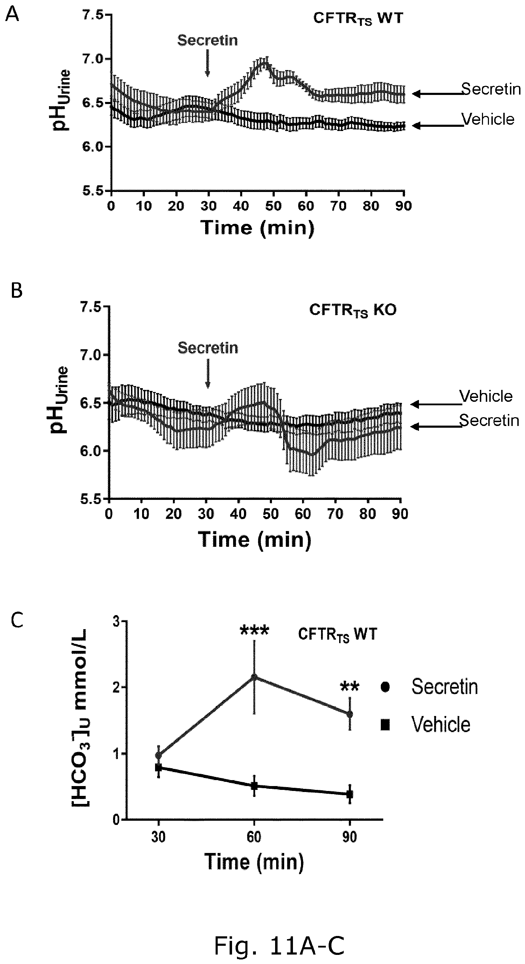

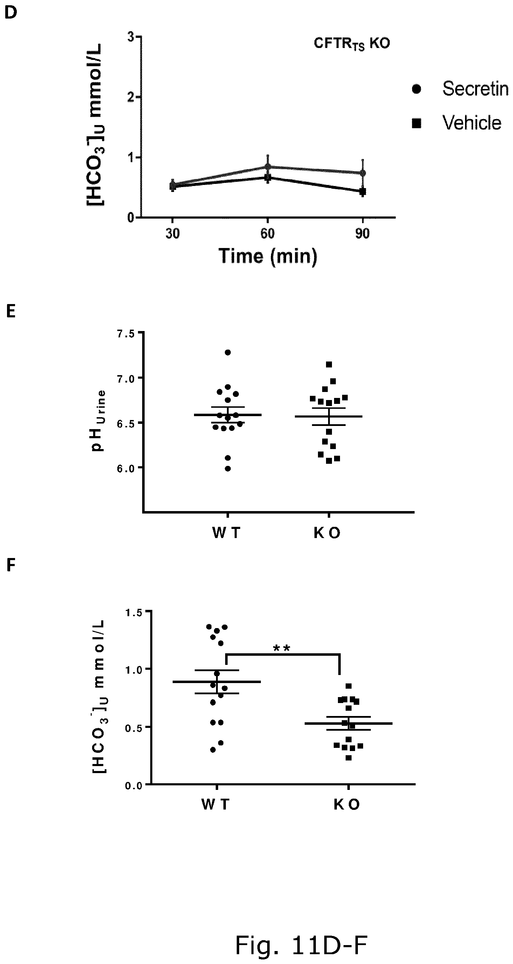

Figure 11 shows that secretin-induced urine alkalisation is absent in tubule specific CFTR KO mice. **(A)** shows urine pH measured in tubule specific CFTR WT with secretin or vehicle injection at the time of the vertical arrow (at 30 min). **(B)** shows urine pH measured in tubule specific CFTR KO with secretin or vehicle injection at the time of the vertical arrow (at 30 min). **(C)** shows amount of bicarbonate measured in tubule specific CFTR WT with secretin or vehicle injection. **(D)** shows amount of bicarbonate measured in tubule specific CFTR KO with secretin or vehicle injection. **(E)** shows baseline measurements of the urine pH in WT and KO tubule specific CFTR mice. **(F)** shows baseline measurements of the amount of bicarbonate in the urine measured in WT and KO tubule specific CFTR mice.

The present invention will now be described in more detail in the following.

### Detailed description of the invention

### Definitions

Prior to discussing the present invention in further details, the following terms and conventions will first be defined:
"Cystic fibrosis" as described herein is also described in short as "CF" and relates to an inherited disease caused by mutation in a single gene being the cystic fibrosis transmembrane conductance regulator (CFTR) gene.
"Bicarbonate" as described herein is to be understood as HCO₃⁻, also known as hydroxidodioxidocarbonate(1-) and hydrogencarbonate.
"Secretin" as described herein is to be understood as the peptide hormone secretin, which is produced in the S cells of the duodenum, which are located in the intestinal glands and was first discovered in 1902 by Bayliss and Staling (Bayliss et al. 1902).
"Subject" as described herein is to be understood as an animal or a human being, said human being including healthy individuals and individuals with CF (i.e. CF patients), where healthy individuals are to be understood as individuals not suffering from CF.
"Compound" as described herein is to be understood as a drug, medicament or other chemical compound, that may be used for treating subjects having CF. This may be approved drugs already in use or new compounds, which are currently being tested or developed for the purpose of treating subjects having CF.
"Effectiveness of a compound against cystic fibrosis" as described herein is to be understood as the potential of a compound to treat CF patients for them to experience less of the symptoms of CF. If the compound is ineffective, it is not capable of lessening the signs and symptoms of the patients.
"Excretion level of bicarbonate" as described herein is defined as the amount of bicarbonate present in the urine i.e. the amount of bicarbonate excreted into the urine by the kidneys. This may be measured as the exact amount of bicarbonate, by the pH, as the total amount of bicarbonate excreted within a specific period or as the excretion rate i.e. the amount of bicarbonate per time period. The excretion level of bicarbonate may further be described as the area under the urine concentration-time curve i.e. AUC (area under the curve), by the Cₘₐₓ or by the Tₘₐₓ.
"AUC_{0-∞}" as described herein is to be understood as the area under the urine concentration-time curve from time 0 and extrapolated to infinity.
"AUC₀₋ₜ" as described herein is to be understood as the area under the urine concentration-time curve from time 0 to time t.
"Cₘₐₓ" as described herein is to be understood as the maximum observed urine concentration in a urine sampling i.e. the peak concentration.
"Tₘₐₓ" as described herein is to be understood as the time at which the peak concentration is observed.
"Bicarbonate gastric load" is to be understood as being obtained by a defined amount of bicarbonate provided as an acute challenge i.e. defined on the basis of obtaining at least a moderate increase in excreted bicarbonate in healthy subjects without inducing noticeable side effects. The acute challenge is thus, only provided as a short-time load, which can be provided as a single dose or several doses consecutively following one another. Preferably, the acute challenge is provided as a single dose. Providing a bicarbonate gastric load would incur an acute challenge for the body to excrete the excessive amount of bicarbonate provided. The amount will be excreted via the kidneys.
"Acute challenge" is to be understood as a dose of bicarbonate, whereby bicarbonate gastric load is obtained.
"A first/second urine sampling" as described herein is to be understood as a single urine sample obtained at a certain point in time, or two or more urine samples obtained over a given time period such as two or more samples obtained over at least three hours.
"A first sample of said first/second urine sampling" as described herein is to be understood as the first urine sample obtained in the first/second urine sampling. Thus, if the urine sampling is only a single sample, the first sample will be this single sample but if the urine sampling is two or more individual samples, the first sample will be the first in time of the two or more individual samples.
"Reference excretion level" as described herein is defined as the excretion level of bicarbonate in the urine prior to the treatment of the subject with bicarbonate.
"Sub-category" as described herein is to be understood as a division of the CF patients into different categories based on the excretion level of bicarbonate in the urine. In one embodiment, the number of sub-categories is three i.e. x1, x2 and x3. x1 is defined as the excretion level of bicarbonate being absent or nearly absent when tested according to the present invention; x2 is defined as a significantly reduced excretion level of bicarbonate when tested according to the present invention and x3 is defined as a normal or almost normal excretion level of bicarbonate when tested according to the present invention.

In this regard "absent" is to be understood as no bicarbonate being excreted.

In this regard "nearly absent" is to be understood as at the most 10%, such as at the most 7%, like at the most 5%, such as at the most 3%, like at the most 2%, such as at the most 1%, like at the most 0.5% of the excretion level observed in healthy individuals. Age-matching may be performed in groups such as 0-15 years, 15-25 years, 25-50 years, 50-65 years, 65+ .

In this regard, "normal" excretion level is to be understood as an excretion level as observed in healthy individuals. Preferably as observed in healthy individuals being age-matched, more preferred the healthy individuals are being age-matched and gender-matched. Age-matching may be performed in groups such as 0-15 years, 15-25 years, 25-50 years, 50-65 years, 65+.

Preferably, the "normal" excretion level is the mean of at least 3 healthy individuals, such as at least 5 healthy individuals, like at least 10 healthy individuals, such as at least 20 healthy individuals, like at least 30 healthy individuals, such as at least 40 healthy individuals, like at least 50 healthy individuals, such as at least 60 healthy individuals, like at least 70 healthy individuals, such as at least 80 healthy individuals, like at least 90 healthy individuals, such as at least 100 healthy individuals.

In this regard, "almost normal" excretion level is to be understood as at least 90%, such as at least 95%, like at least 97%, such as at least 98%, like at least 99%, such as at least 99.9% of the excretion level of bicarbonate observed in healthy individuals. Preferably as observed in healthy individuals being age-matched, more preferred the healthy individuals are being age-matched and gender-matched. Age-matching may be performed in groups such as 0-15 years, 15-25 years, 25-50 years, 50-65 years, 65+

In this regard, "significantly reduced" is to be understood as the levels falling between x1 and x3 i.e. x2 is a group of individuals not belonging to the groups x1 or x3.

"Degree of severity" as described herein is to be understood as the degree to which the CF patients is suffering from the symptoms and health problems caused by CF. It is to be understood that the degree of severity being X1 is to be understood as the CF patient having several clinical symptoms of CF or some of the clinical symptoms but to a severe degree, the degree of severity being X2 is to be understood as the CF patient having some of the clinical symptoms of CF, the degree of severity being X3 is to be understood as the CF patient having only a few of the clinical symptoms.

"Clinical symptoms of CF" as described herein is to be understood as known in the art like one or more of the symptoms described in the following list: lung infections, sinus infections, nasal polyps, pancreatitis, diabetes, liver problems including cirrhosis, infertility, wheezing and shortness of breath, chronic cough with heavy discoloured mucus or blood, swollen belly with gas and discomfort, frequent episodes of diarrhoea, fat-filled stools, poor weight gain, salty frosting on the skin, delayed sexual development, reduced pulmonary function, FEV1 being reduced, mucoid Pseudomonas aeruginosa isolated from airway secretions, hypochloraemic metabolic alkalosis, meconium ileus, protracted jaundice, abdominal or scrotal calcifications, intestinal atresia, persistent infiltrates on chest radiographs, liver disease, allergic bronchopulmonary aspergilloisis.

"Cystic fibrosis mutations" as described herein is to be understood as mutations in the CFTR protein leaving it partially functional, non-functional or expressed in an insufficient amount.

### Effectiveness of drug for treatment

In one aspect, this invention relates to a method of determining the effectiveness of a compound in a subject for treatment of cystic fibrosis comprising the steps of
a. determining a first excretion level of bicarbonate in a first urine sampling obtained from a subject having cystic fibrosis; where said first urine sampling has been obtained prior to said subject has been treated with a compound for treatment of cystic fibrosis;
b. determining a second excretion level of bicarbonate in a second urine sampling from said subject; where said second urine sampling is has been obtained after said subject has been treated with said compound for a predetermined time period;
c. determining the effectiveness of the compound against cystic fibrosis by comparing said first excretion level of bicarbonate to said second excretion level of bicarbonate;
wherein said compound is considered as effective if said second excretion level of bicarbonate is above said first excretion level of bicarbonate and said compound is considered ineffective if said second excretion level of bicarbonate is below or at the same excretion level as said first excretion level of bicarbonate; wherein bicarbonate has been administered to said subject prior to having obtained said first urine sampling and prior to having obtained said second urine sampling; and wherein bicarbonate has been administered to said subject between 0-180 min prior to having obtained a first sample of said first urine sampling and prior to having obtained a first sample of said second urine sampling.

The disclosure further
relates to a method of determining the effectiveness of a compound in a subject for treatment of cystic fibrosis comprising the steps of
a. determining a first excretion level of bicarbonate in a first urine sampling obtained from a subject having cystic fibrosis; where said first urine sampling has been obtained prior to said subject has been treated with a compound for treatment of cystic fibrosis;
b. administering a compound for treatment of cystic fibrosis to said subject;
c. determining a second excretion level of bicarbonate in a second urine sampling from said subject after a predetermined time period;
d. determining the effectiveness of the compound against cystic fibrosis by comparing said first excretion level of bicarbonate to said second excretion level of bicarbonate;
wherein said compound is considered as effective if said second excretion level of bicarbonate is above said first excretion level of bicarbonate and said compound is considered ineffective if said second excretion level of bicarbonate is below or at the same excretion level as said first excretion level of bicarbonate.

Thus, this urine test measures the amount of bicarbonate excreted by the subject in the urine. The first urine sampling is obtained prior to the subject being treated with a compound and the second urine sampling is obtained after a period of treatment. Comparing the excretion levels of bicarbonate will demonstrate whether the compound influences the excretion of bicarbonate into the urine. Thus, it may be established whether or not the compound is effective. If the compound is not effective or not sufficiently effective, the subject may be treated with a different compound. Hence, it can easily be established whether or not a subject benefits from being treated with a certain compound or not.

Preferably, the compound is or has been administered continuously to the subject in the predetermined period e.g. according to the prescription of the compound. Thus, more preferred, the compound is still being administered at the time when the second excretion level is determined.

In a further embodiment, said subject is entered into continuous treatment with said compound if said compound is considered effective.

In a further embodiment, said subject is treated with an alternative compound if said compound tested is considered ineffective.

In a further embodiment, the predetermined time period is at least 1 day, such as at least 2 days, like at least 3 days, such as at least 1 week, like at least 1½ weeks, such as 2 weeks such as at least 3 weeks, like at least 4 weeks, such as at least 6 weeks, like at least 8 weeks.

The subjects have been treated with the compound for a predetermined period in order for the possible effectiveness of the compound to be triggered. Thus, the predetermined period may be different for different compounds.

### Bicarbonate gastric load

In the method bicarbonate has been administered to said subject prior to having obtained said first urine sampling and prior to having obtained said second urine sampling.

In a further embodiment, not being part of the invention, the method further comprises the steps of administering bicarbonate such as a bicarbonate gastric load, to said subject prior to obtaining said first urine sampling and prior to obtaining said second urine sampling. In a still further embodiment, the bicarbonate has been administered as an acute challenge. In an even further embodiment, the acute challenge is an oral challenge i.e. the acute challenge is provided orally.

The bicarbonate, which has been or is administered to the subject prior to obtaining the urine sampling introduces a gastric load in the subject and hereby increases the sensitivity of the urine test. The gastric load is provided as a dose of bicarbonate to provide an acute load of bicarbonate to the subject by means of the acute challenge. The bicarbonate is preferably administered as a single dose i.e. the acute challenge is a single dose. Providing the subject with a gastric load prior to obtaining the urine sampling is demonstrated as shown in the examples to result in an additional excretion of bicarbonate in healthy individuals, who are capable of adjusting the salt homeostasis of the body i.e. an acute renal excretion. In contrast, the CF patients are not able to do so. However, when the CF patients are treated or have been treated with an efficient compound, their adjustment ability will be improved as demonstrated in example 4 and example 5.

The time period between the bicarbonate has been administered and the time of obtaining the first urine sample of the first urine sampling and second urine sampling is preferably of similar length. The length would depend on the set-up of the specific CF urine test to be applied but would normally be of a length, which enables the subject to respond to the bicarbonate.

The bicarbonate has been administered to said subject between 0-180 min prior to obtaining a first sample of said first urine sampling and prior to obtaining a first sample of said second urine , like between 15-240 min, such as between 15-120 min, such as between 30-180 min, like between 30-90 min.

In a further embodiment, the bicarbonate has been administered to said subject at least 30 min prior to obtaining a first sample of said first urine sampling and/or prior to obtaining a first sample of said second urine sampling, such as at least 60 min, like at least 90 min, such as at least 120 min, like at least 150 min, such as at least 180 min.

In a further embodiment, the bicarbonate has been administered to said subject at the most 30 min prior to obtaining a first sample of said first urine sampling and/or prior to obtaining a first sample of said second urine sampling, such as at the most 60 min, like at the most 90 min, such as at the most 120 min, like at the most 150 min, such as at the most 180 min, such as at the most 210 min, like at the most 240 min.

In an embodiment, the bicarbonate has been administered to said subject at around 30 min prior to obtaining a first sample of said first urine sampling and prior to obtaining a first sample of said second urine sampling, such as at around 60 min, like at around 90 min, such as at around 120 min, like at around 150 min, such as at around 180 min.

In a further embodiment, the bicarbonate has been administered to said subject at around 60 min prior to obtaining a first sample of said first urine sampling and prior to obtaining a first sample of said second urine sampling, preferably around 60 min prior to obtaining a first sample of said first and said second urine sampling.

In a further embodiment, the bicarbonate has been administered at an amount of around 10-300 mg/kg body weight, like around 50-150 mg/kg body weight, such as around 60-100 mg/kg body weight, like around 70-90 mg/kg body weight, such as around 75-80 mg/kg body weight.

In one embodiment, the bicarbonate has been administered as a composition comprising bicarbonate. In a further embodiment, the composition comprising bicarbonate does not comprise secretin. The bicarbonate is preferably dissolved in water. In a still further embodiment, the composition consists of bicarbonate dissolved in a fluid such as water.

In one embodiment, the volume of the composition is 100 - 300 ml, such as 200 ml.

### Secretin

In a further embodiment, secretin has been administered to said subject prior to obtaining said first urine sampling and prior to obtaining said second urine sampling. In a further embodiment, the method further comprises the steps of administering secretin to said subject prior to obtaining said first urine sampling and prior to obtaining said second urine sampling.

In an aspect not being part of the invention alternatively or in addition to the gastric load of bicarbonate the subject may be treated with secretin prior to obtaining the first or second urine sampling. Secretin interacts with the secretin receptor (SCTR), which leads via stimulation of the cAMP/PKA signalling pathway to the activation of apical CFTR channel. Thus, secretin would force the excretion level of bicarbonate in the urine sample if the CFTR is functional. An effective drug would thus, result in improved CFTR functioning in the CF patients and thus, an increased secretin-induced excretion level of bicarbonate.

The time period between the secretin has been administered and the time of obtaining the first urine sample of the first urine sampling and/or second urine sampling is preferably of similar length. The length would depend on the set-up of the specific CF urine test to be applied but would normally be of a length, which enables the subject to respond to the secretin.

In one embodiment, the secretin has been administered to said subject between 0-240 min prior to obtaining a first sample of said first urine sampling and/or prior to obtaining a first sample of said second urine sampling, such as between 0-180 min, like between 15-240 min, such as between 15-120 min, like between 30-180 min, such as between 30-90 min, like around 60 min.

In a further embodiment, the secretin has been administered to said subject at least 30 min prior to obtaining a first sample of said first urine sampling and/or prior to obtaining a first sample of said second urine sampling, such as at least 60 min, like at least 90 min, such as at least 120 min, like at least 150 min, such as at least 180 min.

In a further embodiment, the secretin has been administered to said subject at the most 30 min prior to obtaining a first sample of said first urine sampling and/or prior to obtaining a first sample of said second urine sampling, such as at the most 60 min, like at the most 90 min, such as at the most 120 min, like at the most 150 min, such as at the most 180 min, like at the most 210 min, such as at the most 240 min.

In a further embodiment, the secretin is / has been administered to said subject at a concentration of 0.1-1 µg/kg body weight, such as 0.2-0.7 µg/kg body weight, like 0.3-0.5 µg/kg body weight, such as 0.4 µg/kg body weight.

The secretin may be administered by any means, which leaves the hormone functioning. Preferably, secretin is administered as a bolus injection either intravenously (i.v.) or subcutaneously.

In a further embodiment, secretin is or has been administered as a single dose i.e. secretin is not or has not been administered continuously to the subject.

### Reference level

In a further embodiment, the method is further described by
- said first excretion level of bicarbonate in said first urine sampling being adjusted to a first reference excretion level prior to determining the effectiveness of the compound; and/or
- said second excretion level of bicarbonate in said second urine sampling is adjusted to a second reference excretion level prior to determining the effectiveness of the compound.

In a further embodiment, said first reference excretion level is an excretion level of bicarbonate determined in a urine sample from said subject prior to obtaining said first urine sampling and prior to administration of bicarbonate; and said second reference excretion level is an excretion level of bicarbonate determined in a urine sample from said subject prior to obtaining said second urine sampling and prior to administration of bicarbonate.

The first and the second reference excretion level is obtained in order to adjust the first excretion level and the second excretion level to potential deviations in the subjects daily bicarbonate level.

Thus in some embodiments, the first and second reference excretion level is measured in a first and second reference urine sample obtained prior to the subject is or has been administered with the gastric loading of bicarbonate.

The first and second reference urine samples are preferable obtained at the most 1 hour, such as the most 30 min, like at the most 15 min prior to the subject is or has been administered with the gastric loading of bicarbonate.

Preferably, the reference urine sample is obtained just before the subject is or has been administered with the gastric loading of bicarbonate.

Preferably, the time period is similar to the first and second reference urine samples.

### Measurement of the urine samplings

The bicarbonate in the urine samplings may be measured in different ways known in the art. In one embodiment, the level is measured by measuring the amount of bicarbonate excreted using techniques known in the art for measurement of bicarbonate e.g. in blood like a Corning 960 analyser. This embodiment is based on a principle of measuring the carbon dioxide released from the sample during acidification. An example of such a measurement is disclosed in Trepiccione et al., 2017 using a custom built infrared CO2-sensor based system for smaller volume samples as further described in the example section.

Other techniques known in the art for measurement of bicarbonate are based on a principle of colouring.

In a further embodiment, the bicarbonate excretion level is measured by measuring the pH of the urine samplings using standard measurements of pH such as pH-electrodes. Hereby, a more relative measurement of the bicarbonate excretion level is obtained as the pH of the urine sample is related to the amount of bicarbonate but does not provide the exact amount. It is to be understood that the pH will be higher, the higher the bicarbonate concentration of the urine sample. Thus, an increased excretion of bicarbonate in the urine will result in a higher pH.

In a further embodiment, said first urine sampling and/or second urine sampling is two or more individually obtained urine samples. In a still further embodiment, the individually obtained urine samples are merged into an accumulated sample. Preferably, the individual samples are obtained over a certain time period.

Hereby is to be understood that the first urine sampling and/or the second urine sampling may each comprise several single samples obtained at different time points. In one embodiment, the samples are obtained at an hourly rate for up until at least three hours. The individual samples can either be accumulated into one sample for a single measurement or the individual samples may be measured individually showing the bicarbonate excretion level over time. Preferably, the measurement used is identical between the first urine sampling and the second urine sampling.

In one embodiment, the excretion level of bicarbonate is measured as the AUC_{o-∞} In a further embodiment, the excretion level of bicarbonate is measured as the AUC₀₋ₜ. Where "0" is the starting time for the urine test such as the time for obtaining the reference urine sample and/or for administering bicarbonate to the subject. "t" would be any given time for defining one boundary of the area, and would preferably be the end time of the sampling. In some embodiments, "t" would be chosen as any time in the following intervals: 30 min - 8 hrs, 1 hr-6 hr, 2 hr-4 hr. In some embodiments, "t" would be below 8 hrs, such as below 7 hrs, like below 6 hrs, such as below 5 hrs, like below 4 hrs, such as below 3 hours, like below 2 hrs, such as below 1 hr. In some embodiments, "t" would be above 8 hrs, such as above 7 hrs, like above 6 hrs, such as above 5 hrs, like above 4 hrs, such as above 3 hours, like above 2 hrs, such as above 1 hr. In further embodiments, "t" would be around 8 hrs, such as around 7 hrs, like around 6 hrs, such as around 5 hrs, like around 4 hrs, such as around 3 hours, like around 2 hrs, such as around 1 hr. In one embodiment, "t" is 3 hrs.

In a still further embodiment, the excretion level of bicarbonate is measured as the Cₘₐₓ. In a still further embodiment, the excretion level of bicarbonate defined by Tₘₐₓ

In a still further embodiment, the excretion level of bicarbonate is the excretion rate of the bicarbonate defined as the amount of bicarbonate excreted per time period. When the excretion level is demonstrated in a curve i.e. the first and second excretion level is a curve formed by the measurement of several individual samples, the change between the first and second excretion level may be the change in excretion rate shown as a shift in the slopes of the curves.

In a further embodiment, the volume of urine is measured to calculate the total amount of bicarbonate excreted.

Preferably, the subject obtains a liquid after each urine sample if the urine sampling is two or more samples. The liquid is preferably water. In one embodiment, the liquid is 100-350 ml, such as 150-250 ml, like 200 ml.

### Sub-categorizing cystic fibrosis

This disclosure also relates to a method for sub-categorizing cystic fibrosis by
a. determining a first excretion level of bicarbonate in a first urine sampling from a subject having one or more cystic fibrosis mutations;
b. determining whether the first excretion level of bicarbonate belongs to sub-category x1, x2 or x3; wherein x1, x2 and x3 are ranges defining different sub-categories of cystic fibrosis; and
wherein x1 is defined as the excretion level of bicarbonate being absent or nearly absent; x2 is defined as the excretion level of bicarbonate being significantly reduced and x3 is defined as the excretion level of bicarbonate being normal or almost normal.

In one embodiment, the degree of severity is X1 if said first excretion level of bicarbonate belongs to sub-category x1; the degree of severity is X2 if said first excretion level of bicarbonate belongs to sub-category x2, and the degree of severity is X3 if the first excretion level of bicarbonate belongs to sub-category x3.

Sub-categorizing the subjects with one or more cystic fibrosis mutations i.e. CF patients by means of the bicarbonate urine test provides an efficient and simple way of dividing the patients into groups having different clinical symptoms and expected outcome of treatment with specific compounds as well as deciding whether the patients are to be treated or not. Thus, this simple test would be able to categorize the subjects without different mutations of the genes but rather according to the function of CFTR, which is likely to be a complex interplay between the CFTR and the local cellular environment in a given subject.

In one embodiment, bicarbonate has been or is administered to said subject prior to obtaining said first urine sampling. The bicarbonate, which has been or is administered to the subject prior to obtaining the urine sampling introduces a gastric load in the subject and hereby increases the sensitivity of the urine test.

In a further embodiment, the bicarbonate has been or is administered as an acute challenge.

In one embodiment, x1 is at the most 10% of the excretion level observed in healthy individuals, x2 is 11-89% of the excretion level observed in healthy individuals and x3 is at least 90% of the excretion level observed in healthy individuals. Preferably, the percentage calculations are performed based on excretion levels obtained when the urine test is performed under similar conditions in both healthy individuals and CF patients. By similar conditions are to be understood that e.g. the time intervals are similar, the amount of bicarbonate and secretin etc.

In one embodiment, the "normal" excretion level is the peak concentration (Cₘₐₓ). In another embodiment, the "normal" excretion level has a Cₘₐₓ of 30-70 mM, such as 30-60 mM bicarbonate, like 45-65 mM, such as 35-55 mM bicarbonate, like 40-50 mM bicarbonate when providing around 79 mg/kg body weight in 200 ml fluid such as water to healthy individuals.

In one embodiment, the "normal" excretion level is the Area under the curve, preferably AUC₀₋₃. In another embodiment, the "normal" excretion level has a AUC₀₋₃ of 75-125 mmol bicarbonate*t/L, such as 80-120 mmol bicarbonate*t/L, like 90-110 mmol bicarbonate*t/L when providing around 79 mg/kg body weight in 200 ml fluid such as water to healthy individuals.

The period between the time when bicarbonate and/or secretin has been administered and the time of obtaining the first urine sample of the first urine sampling would depend on the set-up of the specific CF urine test to be applied but would normally be of a length, which enables the subject to respond to the bicarbonate and/or secretin.

In one embodiment, the bicarbonate and/or secretin has been administered to said subject between 0-240 min prior to obtaining a first sample of said first urine sampling and/or prior to obtaining a first sample of said second urine sampling, such as between 0-180 min, like between 15-240 min, such as between 15-120 min, like between 30-180 min, such as between 30-90 min.

In a further embodiment, the bicarbonate has been administered to said subject at least 30 min prior to obtaining a first sample of said first urine sampling, such as at least 60 min, like at least 90 min, such as at least 120 min, like at least 150 min, such as at least 180 min.

In a further embodiment, the bicarbonate has been administered to said subject at the most 30 min prior to obtaining a first sample of said first urine sampling, such as at the most 60 min, like at the most 90 min, such as at the most 120 min, like at the most 150 min, such as at the most 180 min, like at the most 210 min, such as at the most 240 min.

In a further embodiment, the bicarbonate has been administered to said subject at around 30 min prior to obtaining a first sample of said first urine sampling, such as at around 60 min, like at around 90 min, such as at around 120 min, like at around 150 min, such as at around 180 min. In a still further embodiment, the bicarbonate is or has been administered to said subject around 60 min prior to obtaining a first sample of said first urine sampling.

In a further embodiment, the bicarbonate has been administered at an amount of around 50-150 mg/kg body weight, such as around 60-100 mg/kg body weight, like around 70-90 mg/kg body weight, such as around 75-80 mg/kg body weight.

In a further embodiment, secretin has been administered to said subject prior to obtaining a first sample of said first urine sampling. The secretin would force the excretion level of bicarbonate in the urine sample if the CFTR is functional. In a further embodiment, secretin has been or is administered as a single dose.

In a further embodiment, the secretin has been administered to said subject at least 30 min prior to obtaining a first sample of said first urine sampling, such as at least 60 min, like at least 90 min, such as at least 120 min, like at least 150 min, such as at least 180 min.

In a further embodiment, the secretin has been administered to said subject at the most 30 min prior to obtaining a first sample of said first urine sampling, such as at the most 60 min, like at the most 90 min, such as at the most 120 min, like at the most 150 min, such as at the most 180 min, like at the most 210 min, such as at the most 240 min, like around 60 min.

In one embodiment, said subject is entered into a drug treatment if said subject is defined in sub-category x1 or sub-category x2 and wherein said subject is left untreated if the subject is defined as belonging to sub-category x3.

### Use of bicarbonate urine test in relation to cystic fibrosis

In a further aspect, the use of the excretion level of bicarbonate in the urine to determine the effectiveness of a compound against cystic fibrosis is described. Hereby, one may easily circumvent the issue that different CF patients react differently to the same compounds even though they carry similar mutations, in a simple and efficient manner by using the urine test as described herein.

In a further aspect, the use of a composition comprising bicarbonate for diagnosing cystic fibrosis is described. In a still further aspect, a composition comprising bicarbonate for use in diagnosing cystic fibrosis is described. In an embodiment, the composition is an acute challenge inducing a gastric load. In a further embodiment, the acute challenge is a single dose of a composition comprising bicarbonate inducing a bicarbonate gastric load.

Hereby, it is to be understood that a composition comprising bicarbonate is used to introduce a gastric load in a subject having cystic fibrosis and that the excretion of bicarbonate will be lower or delayed compared to the level observed from a healthy subject. The acute challenge is preferably provided as a single load i.e. a one time dose in order for an acute renal excretion to be observed. Hereby, cystic fibrosis can be diagnosed in a subject.

The composition may be any non-toxic bicarbonate allowable for human consumption such as sodium bicarbonate, potassium bicarbonate, magnesium bicarbonate and calcium bicarbonate.

The concentration of the composition is preferably between 5-1000 mM, such as 7-100 mM, like 8-50 mM, such as 9-25 mM. Preferably, the concentration of the composition is 13 mmol/L for a 70 kg person.

In a further embodiment, the composition comprising bicarbonate is a solution or a bolus, preferably a solution.

In a further embodiment, the composition is orally administered. Alternatively, the composition may be administered intravenously (i.v.).

The disclosure will now be described in further details in the following non-limiting examples.

### Examples

### Example 1 - Materials and methods

### A. Secretin

### Animals

The experiments were performed in littermate mice bred from heterozygotes. Mice of both genders were used at an approximate age of 2 to 4 months.

The Pendrin KO mouse was produced by insertion of a neo-cassette replacing exon 8 in the Scl26a4 gene, leading to loss of pendrin expression as described in (Everett et al. 2001). It was purchased from The Jackson Laboratory, JAX stock #018424.

The global CFTR (CFTR_{G}) KO mouse (CFTR S489X-; FABP-hCFTR) was purchased from The Jackson Laboratory, JAX stock #002364. This is a transgenic global CFTR KO mouse strain with expression of human CFTR (hCFTR) under the promoter activity of rat fatty acid binding protein 2, intestinal (Fabp2) promoter, which corrects the otherwise lethal intestinal phenotype in CFTR KO mice (Zhou et al. 1994).

The Renal tubule specific CFTR (CFTR_{TS}) KO mouse was produced in the laboratory of Dr. Kunzelmann and Dr. Schreiber in Regensburg/Germany and performed in compliance with the guidelines for the welfare of experimental animals issued by the Federal Government of Germany. C57BL6/J CFTR fl10 mice (Hodges et al. 2008) were bred in a C57Bl/6J background generated by the Cre/loxP-system with tissue specific Cre-recombinase expression from the Pax8 locus (Kos 2004; Bouchard et al. 2004).

There were no significant differences between control, intervention, KO or WT groups regarding weight and age in each experimental series.

### Immunohistochemistry

Mouse kidneys were fixed by perfusion with 4% paraformaldehyde and post-fixed in 0.5 mol/l sucrose and 4% paraformaldehyde solution. Cryosections of 5 µm were incubated in 0.1% sodium dodecyl sulfate for 5 min, washed with PBS, and blocked with 5% bovine serum albumin (BSA) and 0.04% Triton X-100 in PBS for 30 min. Sections were incubated with primary antibodies against CFTR H-182 (rabbit IgG, Santa Cruz Biotech) and Pendrin E-20 (goat IgG, Santa Cruz Biotech) in 0.5% BSA and 0.04% Triton X-100 overnight at 4 °C and with anti-rabbit Alexa488 IgG or antigoat Alexa546 (Invitrogen, Darmstadt, Germany) for 1h at 37°C. Sections were counterstained with Hoe33342 (Sigma-Aldrich). Immunofluorescence was detected using an Axiovert 200 microscope equipped with ApoTome and AxioVision (Zeiss, Germany).

### In vivo urine pH measurements in mice

Induction of anaesthesia was performed with i.p. injection of 0.1 ml/ g BW of a ketamine-xylazine mix (10 mg/ml and 1 mg/ml). An i.v. access was obtained by cannulation of one tail vein and the animals were kept in anaesthesia by a continuous rate infusion (CRI) corresponding to half of the induction dose pr. hour. Mice were infused with a solution containing 145 mM NaCl at pH 7.4 with a perfusion rate of 0.1 ml h⁻¹ via a syringe pump (Model 33 Twin syringe pump, Harvard Apparatus, USA).

Bladder catheterization was introduced and urine was collected every 5 minutes. Urinary pH was measured continuously throughout the experiment by placement of a micro pH electrode (pH-200, Unisense, Aarhus, DK) in the outflow of the catheter and the reference electrode was placed into the suprapubic operation wound (see fig. 7A). Following a 30-min. control period, mice were injected intraperitoneally with either secretin (10µg/25g BW) or saline.

### Urine HCO₃⁻ measurements in mice

The urine was collected and pooled for 30 minutes allowing measurement of mean [HCO₃⁻] in the time periods 0-30, 30-60 and 60-90 after starting the experiment. In experiments with too low urine output, urine was pooled to allow analysis of [HCO₃⁻] before and after secretin application. Measurements were performed by a Corning 960 analyser (Savory et al. 1978) and calibrated to small volume samples of 50µl or alternatively by a custom built infrared COz-sensor based system for smaller volume samples (Trepiccione et al. 2017) (10-40µl).

### Statistics

All values are presented as mean values ± standard error of mean (SEM). Due to a low number of observations in each series, n=7 in most, we were not able to determine whether the data followed a Gaussian distribution. Therefore, two-tailed Wilcoxon matched pairs signed rank test, in paired data, and two-tailed Mann-Whitney test, in unpaired data, were performed to test for significance. A p-value≤0.5 was chosen to represent significance. *, ** and *** indicate p-values of respectively ≤ 0.05, ≤ 0.01 and ≤ 0.001.

### B. Bicarbonate gastric load

### Bicarbonate oral challenge

### Mice

Awake mice (animals as described under section A.) were manually restrained and a gavage needle was inserted into their stomach. A dose of 60 µmoles NaHCO₃ /25 g bodyweight in 400 µl of water was then instilled. Mice were then placed in metabolic cages and their urine collected for further measurement.

Urine was sampled and accumulated for 3 hr before measurement. Bicarbonate and pH were measured as described under section A.

### Human

This was performed according to the following protocol
- Participants met fasting but was allowed to drink water and void at home.
- 79 mg/kg BW NaHCO₃ (CAS Number: 144-55-7, Sigma Aldrich, purity: ≥99.7%, Ord. nr. S5761) was dissolved in 200 ml tap water (0.94 mmol/kg BW i.e. 65 mmol for a 70 kg subject.
- The participants were not allowed to drink, eat or spontaneous void during the experiment.
- Volume was measured for each voiding sample. 2x 10 ml samples from each voiding sample was frozen directly after collection for later measurement of the bicarbonate excretion level.

A baseline urine sample (reference level) was obtained before drinking the bicarbonate composition or tap water (as a vehicle). After 1 hr. urine samples were collected and the participant drank 200 ml of tap water. One hour later, urine samples were collected again and the participant drank further 200 ml of tap water. After three hours from the baseline the final urine sample were collected and the participants drank another 200 ml of tap water.

### Selection of CF patients

Nine adult CF patients aged 19 to 45 of both genders with the homozygote mutation ΔF508 were signed up for this test.

### Selection of human volunteers

Twelve healthy adult volunteers aged 22 to 59 years of both genders were tested. The volunteers were healthy and without any known CFTR protein mutations.

### Treatment with drug modulator

Three of the adult CF patients were treated with Orkambi^{®} (400 mg lumacaftor/ 200 mg ivacaftor twice a day) for four weeks before testing the CF patients for a second time. These included patients of both genders between the age of 19 and 45.

Six of the adult CF patients were treated with Symkevi^{®} (100 mg tezacaftor and 150 mg ivacaftor in the morning and 150 mg ivacaftor in the evening) for four weeks before testing the CF patients for a second time. These included patients of both genders between the age of 20 and 40.

### Measurement of bicarbonate in the urine

Five ml of urine were given into a 250 ml glass flask. Using excess acid addition (5 ml 1 N HCl) all HCO₃⁻ was released from the fluid phase and CO₂ was measured with an infrared CO₂-sensor (Trepiccione et al. 2017).

### Statistics

A two-tailed paired t-test was used to test for significance. A p-value≤0.5 was chosen to represent significance.

### Example 2 - Acute renal excretion of bicarbonate following an oral challenge requires CFTR and pendrin

### Aim

To study the effect in mouse after oral challenge with bicarbonate (bicarbonate gastric load) on the excretion of bicarbonate in the urine.

### Results

Following oral dose of bicarbonate to WT mice as well as CFTR and pendrin knock-out mice via gastric loading (fig. 1A), the bicarbonate concentration in the urine and the pH of the urine was measured.

Urine bicarbonate concentration was measured in a cumulative sample after 3 hours and found to increase as well as the pH of the urine in WT mice (fig. 1B and fig. 1C).

Mice treated with bicarbonate are labelled "NaHCO₃" and vehicle treated mice are labelled "H₂O". Untreated mice are labelled "-" in fig. 1B.

As shown in fig. 1B and fig. 1C, mice lacking CFTR are unable to excrete the ingested amount of bicarbonate. The same results were obtained in pendrin knock-out mice (data not shown).

### Conclusion

Thus, these results show that CFTR is important for the acute renal excretion of a defined amount of orally ingested bicarbonate.

### Example 3 - Monitoring of the function of CFTR in humans by CF urine test

### Aim

To measure the functioning of CFTR and the influence of a CF urine test, which introduces a gastric load of bicarbonate.

### Results

The protocol of the applied CF urine test is schematically shown in fig. 2.

At t=0, a base-line urine sample was obtained for determining a first reference level of bicarbonate. Immediately hereafter or just before, the subjects were treated with bicarbonate to induce a gastric load hereof or tap water (for reference measurement). In addition, the venous acid-base status was checked.

At t= 1 hr, the subjects were to void the first sample. The volume hereof was noted and the concentration of bicarbonate measured. The subjects then drank 200 mL of tap water. In addition, the venous acid-base status was checked.

At t=2hrs, the subjects were to void the second sample. The volume hereof was noted and the concentration of bicarbonate measured. The subjects then drank 200 mL of tap water.

At t=3hrs, the subjects were to void the third sample. The volume hereof was noted and the concentration of bicarbonate measured. The subjects then drank 200 mL of tap water.

In fig. 3 the results of the CF urine test is shown for eight healthy adult volunteers. A fast and large increase in [HCO₃⁻]ᵤ is seen after 1 h that peaks at 2 hours (Tₘₐₓ) after oral ingestion of bicarbonate having a Cₘₐₓ of around 45-65 mM. The corresponding water drinking control experiments in the same volunteers shows no changes in [HCO₃⁻]ᵤ.

The test was also applied in nine adult CF patients with the homozygote mutation of ΔF508 in CFTR. Fig. 4 demonstrates that the [HCO₃⁻]ᵤ increases were significantly attenuated in the nine adult CF patients as compared to twelve healthy controls. The results indicate that CF patients (ΔF508/ΔF508) have a reduced ability to acutely excrete a defined oral bicarbonate load showing a Cₘₐₓ of around 14-28 mM at a Tₘₐₓ of 2 hrs.

### Conclusion

These results demonstrate that healthy adults respond quickly to the CF urine test and the introduction of a gastric load of bicarbonate by fast increase in the amount of bicarbonate excreted. In contrast, CF patients having a mutated CFTR gene show an attenuated response to the urine test.

### Example 4 - Quantification of effectiveness of CFTR modulator drugs in CF patients

### Aim

To quantify the effectiveness of CFTR modulator drugs in CF patients.

### Results

Three CF patients having the same mutation in the CFTR gene (ΔF508/ΔF508) where tested with the bicarbonate drinking test (CF urine test) before and after 4 weeks of treatment with Orkambi^{®}.

The results of three patients are shown in fig. 5. Fig. 5A-C demonstrate the result per patient, while fig. 5D is an average of the treatment of the three patients. The dotted line illustrates the result of the test prior to treatment with Orkambi^{®}, while the full line illustrates the result of the test after treatment with Orkambi^{®}. The vertical arrow shows the difference before and after treatment with Orkambi^{®} at Tₘₐₓ.

The area under the curve for the three hours (AUC₀₋₃) was calculated based on the measurements behind Figures 4 and 5A-D resulting in Figure 5E demonstrating the mean and standard deviation of the results. This figure clearly demonstrates that bicarbonate is excreted to a higher extend by the controls (AUC₀₋₃in the range 85-123 mmol bicarbonate *t/L) than by the CF patients both before (AUC₀₋₃in the range 14-58 mmol bicarbonate *t/L) and after treatment with Orkambi^{®} (AUC₀₋₃in the range 37-89 mmol bicarbonate *t/L). However, the results also demonstrate that the patients are responding to the Orkambi^{®} treatment by excreting more bicarbonate after treatment.

In all CF patients a marked increase in [HCO₃⁻]ᵤ was measured after the initiation of Orkambi^{®} treatment. These results show that the CF urine test documents an increased urinary excretion of bicarbonate following a gastric load after the treatment of CF patients with Orkambi^{®}.

### Conclusion

It is demonstrated that the effect of Orkambi^{®} enables the patients to increase the amount of bicarbonate excreted following the gastric load of bicarbonate in the urine test. In addition, the results demonstrate that the patients react differently to the treatment.

### Example 5 - Quantification of effectiveness of CFTR modulator drugs in CF patients

### Aim

To quantify the effectiveness of CFTR modulator drugs in CF patients.

### Results

Six CF patients having the same mutation in the CFTR gene (ΔF508/ΔF508) where tested with the bicarbonate drinking test (CF urine test) before and after 4 weeks of treatment with Symkevi^{®}.

The results of six patients are shown in fig. 6. Fig. 6A-F demonstrate the result per patient, while fig. 6G is an average of the treatment of the six patients. The dotted line illustrates the result of the test prior to treatment with Symkevi^{®}, while the full line illustrates the result of the test after treatment with Symkevi^{®}. The peak value (t=2hrs) for pre-symkevi demonstrated a range from 11-28 mM while the peak value (t=2 hrs) for post-symkevi was in the range from 17-26 mM.

Fig. 6H demonstrates a mean of the results obtained in 6A-F calculated as the AUC₀₋₃ of excreted bicarbonate. The range of the results obtained was 22-45 mmol bicarbonate*t/L pre-symkevi while it was 27-38 mmol bicarbonate*t/L post-symkevi.

The results show a highly individual response of the CF patients to Symkevi^{®} treatment showing either an increased effect, no effect or a decreased effect on the excretion of bicarbonate following a gastric load.

### Conclusion

It is demonstrated that the CF urine test is able to easily detect differences between the response of individual CF patients to the modulator drug. In addition, the results demonstrate that the patients react differently to the treatment and indicate that treatment with Symkevi^{®} may not be as effective in all CF patients. Thus, it is demonstrated that this simple test easily distinguish CF patients that would benefit from Symkevi^{®} treatment or not.

### Example 6 - Secretin-induced urinary bicarbonate excretion

### Aim

The aim of the present invention was to study the secretin-induced urinary HCO₃⁻excretion.

### Method

This was studied in different mouse models as described in further detail in Example 1.

### Results

### Immunolocalisation of the SCTR and CFTR to pendrin positive cells

Immunhistochemistry staining of pendrin was confirmed in the apical membrane domains of the cortical collecting ducts (CCD). In pendrin positive cells of the CCDs SCTR staining could be detected on the contra-lateral side (fig. 7A). In panel fig. 7B, the pendrin positive cells were also co-stained with an antibody against CFTR. CFTR staining decorated the luminal membrane domain of some pendrin positive cells and also stained other cellular regions of CCD cells. These results indicate that the SCTR localizes to the basolateral side of β-IC of the CCD and that CFTR localizes to the same membrane domain as pendrin in β-IC.

### Secretin induces an acute urinary alkalisation

Urinary pH (pHᵤ) in anaesthetized pendrin WT mice was monitored for a 30 min baseline period before i.p. application of an equal saline volume (100 µL) containing either 10µg/25 g BW secretin or just saline. In fig. 8B two original pHᵤ traces are shown, one with injection of 10 µg of secretin and one control trace. Within a few minutes after application of secretin (vertical arrow), an acute and robust urinary alkalization was observed. Maximal urine alkalisation was observed between 20 to 30 min after secretin application (see also fig. 9, 10 and 11). Subsequently, the dose dependency of this secretin effect was studied. A significant increase of pHᵤ was observed with a dose of 0.1 µg. Higher doses of 1, 5 and 10 µg secretin (i.p.) caused increasing pHᵤ alkalisations. At the highest dose of 10 µg/ 25 g BW, the peak pHᵤ increase in fig. 8C amounted to ~0.38 units. In all pursuing experiments, the dose of 10 µg/ 25 g BW was used. These results indicate that secretin causes an acute and dose dependent urinary alkalisation.

### The secretin-induced urine alkalisation is absent in pendrin KO mice

The effect of secretin on pHᵤ in pendrin WT and KO mice was studied. Fig. 9A shows that secretin treated WT animals respond after a lag time of approximately 5 minutes with a marked transient urinary alkalization reaching peak mean alkalisations after some 25 minutes of 0.397 ± 0.1 pH units (p=0.0019, n=7). Vehicle injected mice showed no response. Importantly, no significant pHᵤ alkalisations were observed in pendrin KO (fig. 9B). Somewhat surprisingly, in pendrin KO mice secretin induced a urinary acidification starting after some 20 minutes post-injection. A urinary acidification effect was never observed in pendrin WT mice. Secretin treated WT mice had significantly higher urinary [HCO₃⁻] compared to vehicle treated in the 60 minutes following injection with a mean of 1.08 ± 0.3 mM vs 0.1697 ± 0.1 mM in controls (fig. 9C, p=0.0082, n=6-7). No differences were observed in KO mice (n=7, fig. 9D). The urine HCO₃⁻ excretion rate was significantly increased in secretin-treated WT mice after 60 minutes post secretin injection as compared to vehicle treated with a mean of 5.61 ± 2.06 vs 1.18 ± 0.57 nmol/h/g BW (p=0.0221, n=6-7). No differences in urinary HCO₃⁻ excretion were observed in KO mice (data not shown). KO mice had a more acidic urine at baseline as compared to WT with a mean difference of pHᵤ of 0.26 ± 0.17 (p=0.02, n=14, fig. 9E). KO mice also had a tentatively lower urinary [HCO₃⁻] than WT, though not reaching a level of significance (fig. 9F). These results illustrate the absolute pendrin dependence of secretin-inducible urine HCO₃⁻ excretion.

### The secretin-induced urine alkalisation is absent in global CFTR KO mice

Subsequently, the effect of secretin on pHᵤ in global CFTR WT and KO mice was studied. Fig. 10A shows that secretin treated WT animals respond after a lag time of a few minutes with a marked transient urinary alkalization lasting about >35 minutes and reaching peak mean alkalisations of 0.53 ± 0.24 pH units, (p=0.03, n=6). Vehicle injected CFTR WT mice showed no response. In CFTR KO mice, the secretin effect urinary pH was virtually absent (fig. 10B). A small, but significant increase of pHᵤ was found in secretin-treated CFTR KO mice with a peak difference 0.13 pH ± 0.11 units (peak post 20 min. secretin, p=0.03, n=7), as compared to pH prior to treatment, though not differing from vehicle treated KO mice. Vehicle injected CFTR KO mice showed no response.

Upon secretin application urinary [HCO₃⁻] increased markedly in CFTR WT mice and was significantly elevated as compared to the control group (Δ 30 minutes: 3.02 ± 1.06 mM, p=0.022, fig. 10C). The urine HCO₃⁻ excretion rate was increased in WT animals compared to control after 60 min, though not reaching significance(Δ HCO₃⁻excretion rate: 5.55 ± 3.25 nmol/h/g BW (p=0.09, data not shown). No significant differences were observed in CFTR KO-mice, neither comparing to starting or control HCO₃⁻ excretion rate and urine [HCO₃⁻] concentration, at any point during the experiment (fig. 10D). Interestingly, resting urinary [HCO₃⁻] and urinary HCO₃⁻excretion rate were significantly higher in WT- as compared to CFTR KO-mice (fig. 10F). Baseline urine pH in CFTR WT and KO mice were not different (fig. 10E). These results demonstrate that also intact CFTR is necessary to trigger full urine HCO₃⁻excretion. A small residual secretin-induced urine HCO₃⁻ excretion prevails in global CFTR KO mice.

### The secretin-induced urine alkalisation is absent in tubule specific CFTR KO mice

The above data strongly support the conclusion that the absence of renal CFTR is responsible for the inability to excrete urine HCO₃⁻ after secretin application. To test this hypothesis further, the effect of secretin on pHᵤ in tubule specific CFTR (CFTR_{TS}) WT and KO mice were studied. Fig. 11A shows that secretin treated WT animals responded after a lag time of some minutes with a marked transient urinary alkalization reaching peak mean alkalisations of 0.6518 ± 0.11 (p=0.0006, n=7) after about 18 minutes. Vehicle injected CFTR WT mice showed no response. In the CFTR_{TS} KO mice, no significant effect of secretin was found when compared to control experiments (fig. 11B).

Urine HCO₃⁻ measurements were performed on urine samples pooled into 30 minute intervals and revealed a significantly lower urinary [HCO₃⁻] in CFTR_{TS} KO mice during the first 30 minutes of the experiment compared to WT animals with a mean difference of 0.3584 mM (p=0.0079, n=14, fig. 11F). A pronounced increase in urine [HCO₃⁻] occurred in WT animals after secretin administration. The [HCO₃⁻] elevation was highest in the first 30 minutes following secretin administration, but stayed significantly higher compared to controls for the rest of the experiment. Comparing to baseline, urine [HCO₃⁻] revealed a significant increase 60 minutes after administration. Likewise, a significant increase in the urine HCO₃⁻ excretion rate was observed 60 minutes after secretin treatment with a mean difference of 5.623 ± 2.2 nmol/h/kg as compared to baseline (data not shown). No significant secretin-induced differences, either in urine [HCO₃⁻] or urine HCO₃⁻ excretion rate, were found in CFTR_{TS} KO-mice. Baseline urine pH in CFTR_{TS} KO mice were not different to those in WT mice. These data prove that renal CFTR is necessary to permit secretin's action of increasing urine HCO₃⁻ excretion.

### Conclusion

The above results show that mice having a defective pendrin or CFTR are not able to regulate bicarbonate excretion in the urine and consequently, the pH of the urine upon stimulation with secretin does not increase.

### References

Bayliss WM, and Starling EH. "The mechanism of pancreatic secretion." J Physiol. 28(5):325-53, 1902
Bouchard M, Souabni A, and Busslinger M. "Tissue-specific expression of cre recombinase from the Pax8 locus." Genesis 38(3): 105-9, 2004
Everett LA, Belyantseva IA, Noben-Trauth K, Cantos R, Chen A, Thakkar SI, et al. "Targeted disruption of mouse Pds provides insight about the inner-ear defects encountered in Pendred syndrome." Hum Mol Genet. 10(2): 153-61, 2001
Hodges CA, Cotton CU, Palmert MR, and Drumm ML. "Generation of a conditional null allele for Cftr in mice." Genesis 46(10):546-52, 2008
Jentsch, TJ, Stein, V, Weinreich, F, Zdebik, AA: "Molecular structure and physiological function of chloride channels." Physiol Rev, 82: 503-568, 2002
Kos CH. "Cre/loxP system for generating tissue-specific knockout mouse models." Nutr Rev. 62(6 Pt 1):243-6, 2004
Meeting report: "Classification of cystic fibrosis and related disorders." Journal of Cystic Fibrosis 1: 5-8, 2002
O'Sullivan, BP, Freedman, SD: "Cystic fibrosis." Lancet, 373: 1891-1904, 2009
Savory DJ, and Pryce JD. "Quality control of measurements of pH, carbon dioxide tension, and total carbon dioxide in plasma." Clin Chem. 24(9): 1618-9, 1978
Trepiccione F, Iena FM, Catalini L, Carpi FM, Koed M, and Frische S. "Measurement of total CO2 in microliter samples of urine and other biological fluids using infrared detection of CO2." Pflugers Arch. 469(10): 1267-75, 2017.
Zhou L, Dey CR, Wert SE, DuVall MD, Frizzell RA, and Whitsett JA. "Correction of lethal intestinal defect in a mouse model of cystic fibrosis by human CFTR." Science 266(5191):1705-8, 1994

## Claims

1. A method of determining the effectiveness of a compound in a subject for treatment of cystic fibrosis comprising the steps of
a. determining a first excretion level of bicarbonate in a first urine sampling obtained from a subject having cystic fibrosis; where said first urine sampling has been obtained prior to said subject has been treated with a compound for treatment of cystic fibrosis;
b. determining a second excretion level of bicarbonate in a second urine sampling from said subject; where said second urine sampling has been obtained after said subject has been treated with said compound for a predetermined time period;
c. determining the effectiveness of the compound against cystic fibrosis by comparing said first excretion level of bicarbonate to said second excretion level of bicarbonate;
wherein said compound is considered as effective if said second excretion level of bicarbonate is above said first excretion level of bicarbonate and said compound is considered ineffective if said second excretion level of bicarbonate is below or at the same excretion level as said first excretion level of bicarbonate;
wherein bicarbonate has been administered to said subject prior to having obtained said first urine sampling and prior to having obtained said second urine sampling; and
wherein bicarbonate has been administered to said subject between 0-180 min prior to having obtained a first sample of said first urine sampling and prior to having obtained a first sample of said second urine sampling.

2. The method according to claim 1, wherein the bicarbonate has been administered as an oral challenge.

3. The method according to any of the preceding claims, the method is further described by
- said first excretion level of bicarbonate in said first urine sampling being adjusted to a first reference excretion level prior to determining the effectiveness of the compound; and/or
- said second excretion level of bicarbonate in said second urine sampling being adjusted to a second reference excretion level prior to determining the effectiveness of the compound.

4. The method according to claim 3, wherein said first reference excretion level is an excretion level of bicarbonate determined in a urine sample from said subject prior to obtaining said first urine sampling and prior to administration of bicarbonate ; and said second reference excretion level is an excretion level of bicarbonate determined in a urine sample from said subject prior to obtaining said second urine sampling and prior to administration of bicarbonate.

5. The method according to any of the preceding claims, wherein the predetermined time period is at least 2 weeks such as at least 3 weeks, like at least 4 weeks.

6. The method according to any of the preceding claims, wherein said first and/or second excretion level of bicarbonate is measured as the AUC₀₋ₜ.

7. The method according to any of the preceding claims, wherein the bicarbonate has been administered to said subject around 30 min prior to obtaining a first sample of said first urine sampling and prior to obtaining a first sample of said second urine sampling, such as around 60 min, like around 90 min, such as around 120 min, like around 150 min, such as around 180 min.

8. The method according to any of the preceding claims, wherein the bicarbonate has been administered at an amount of 50-150 mg/kg, such as 60-100 mg/kg, like 70-90 mg/kg, such as 75-80 mg/kg.

9. The method according to any of the preceding claims, wherein said first urine sampling and/or second urine sampling is a number of individually obtained urine samples.

10. Use of the excretion level of bicarbonate in the urine to determine the effectiveness of a compound against cystic fibrosis using a method as described in any one of the claims 1-9.

## Patentansprüche

1. Verfahren zum Bestimmen der Wirksamkeit einer Verbindung in einem Patienten zur Behandlung von Mukoviszidose, umfassend die folgenden Schritte:
a. Bestimmen eines ersten Ausscheidungsgrades von Bicarbonat in einer ersten Urinprobenahme, die von einem Patienten mit Mukoviszidose erhalten wurde; wobei die erste Urinprobenahme erhalten wurde, bevor der Patient mit einer Verbindung zur Behandlung von Mukoviszidose behandelt wurde;
b. Bestimmen eines zweiten Ausscheidungsgrades von Bicarbonat in einer zweiten Urinprobenahme von dem Patienten; wobei die zweite Urinprobenahme erhalten wurde, nachdem der Patient für einen vorbestimmten Zeitraum mit der Verbindung behandelt wurde;
c. Bestimmen der Wirksamkeit der Verbindung gegen Mukoviszidose durch Vergleichen des ersten Ausscheidungsgrades von Bicarbonat mit dem zweiten Ausscheidungsgrad von Bicarbonat;
wobei die Verbindung als wirksam angesehen wird, wenn der zweite Ausscheidungsgrad von Bicarbonat über dem ersten Ausscheidungsgrad von Bicarbonat liegt, und die Verbindung als unwirksam angesehen wird, wenn der zweite Ausscheidungsgrad von Bicarbonat unter oder auf dem gleichen Ausscheidungsgrad wie der erste Ausscheidungsgrad von Bicarbonat liegt;
wobei Bicarbonat dem Patienten verabreicht wurde, bevor die erste Urinprobenahme erhalten wurde und bevor die zweite Urinprobenahme erhalten wurde; und
wobei Bicarbonat dem Patienten zwischen 0 und 180 min vor einem Erhalten einer ersten Probe der ersten Urinprobenahme und vor einem Erhalten einer ersten Probe der zweiten Urinprobenahme verabreicht wurde.

2. Verfahren nach Anspruch 1, wobei das Bicarbonat als eine orale Provokation verabreicht wurde.

3. Verfahren nach einem der vorhergehenden Ansprüche, wobei das Verfahren ferner durch Folgendes beschrieben wird:
- der erste Ausscheidungsgrad von Bicarbonat in der ersten Urinprobenahme wird auf einen ersten Referenzausscheidungsgrad eingestellt, bevor die Wirksamkeit der Verbindung bestimmt wird; und/oder
- der zweite Ausscheidungsgrad von Bicarbonat in der zweiten Urinprobenahme wird auf einen zweiten Referenzausscheidungsgrad eingestellt, bevor die Wirksamkeit der Verbindung bestimmt wird.

4. Verfahren nach Anspruch 3, wobei der erste Referenzausscheidungsgrad ein Ausscheidungsgrad von Bicarbonat ist, der in einer Urinprobenahme von dem Patienten vor einem Erhalten der ersten Urinprobe und vor einer Verabreichung von Bicarbonat bestimmt wird; und der zweite Referenzausscheidungsgrad ein Ausscheidungsgrad von Bicarbonat ist, der in einer Urinprobenahme von dem Patienten vor einem Erhalten der zweiten Urinprobe und vor einer Verabreichung von Bicarbonat bestimmt wird.

5. Verfahren nach einem der vorhergehenden Ansprüche, wobei die vorbestimmte Zeitdauer mindestens 2 Wochen beträgt, wie etwa mindestens 3 Wochen, wie mindestens 4 Wochen.

6. Verfahren nach einem der vorhergehenden Ansprüche, wobei der erste und/oder der zweite Ausscheidungsgrad von Bicarbonat als die AUC₀₋ₜ gemessen wird.

7. Verfahren nach einem der vorhergehenden Ansprüche, wobei das Bicarbonat dem Patienten etwa 30 min, wie etwa 60 min, wie etwa 90 min, wie etwa 120 min, wie etwa 150 min, wie etwa 180 min, vor einem Erhalten einer ersten Probe der ersten Urinprobenahme und vor einem Erhalten einer ersten Probe der zweiten Urinprobenahme verabreicht wurde.

8. Verfahren nach einem der vorhergehenden Ansprüche, wobei das Bicarbonat in einer Menge von 50-150 mg/kg, wie etwa 60-100 mg/kg, wie 70-90 mg/kg, wie etwa 75-80 mg/kg, verabreicht wurde.

9. Verfahren nach einem der vorhergehenden Ansprüche, wobei die erste Urinprobenahme und/oder die zweite Urinprobenahme eine Anzahl von individuell erhaltenen Urinproben ist.

10. Verwendung des Ausscheidungsgrades von Bicarbonat im Urin zum Bestimmen der Wirksamkeit einer Verbindung gegen Mukoviszidose unter Verwendung eines Verfahrens nach einem der Ansprüche 1-9.

## Revendications

1. Procédé de détermination de l'efficacité d'un composé chez un sujet pour le traitement de la mucoviscidose comprenant les étapes de
a. détermination d'un premier niveau d'excrétion de bicarbonate dans un premier échantillonnage d'urine obtenu à partir d'un sujet atteint de mucoviscidose ; où ledit premier échantillonnage d'urine a été obtenu avant que ledit sujet ait été traité avec un composé pour le traitement de la mucoviscidose ;
b. détermination d'un second niveau d'excrétion de bicarbonate dans un second échantillonnage d'urine dudit sujet ; où ledit second échantillonnage d'urine a été obtenu après que ledit sujet a été traité avec ledit composé pendant une période de temps prédéfinie ;
c. détermination de l'efficacité du composé contre la mucoviscidose en comparant ledit premier niveau d'excrétion de bicarbonate audit second niveau d'excrétion de bicarbonate ;
ledit composé étant considéré comme efficace si ledit second niveau d'excrétion de bicarbonate est supérieur audit premier niveau d'excrétion de bicarbonate et ledit composé étant considéré comme inefficace si ledit second niveau d'excrétion de bicarbonate est inférieur ou au même niveau d'excrétion que ledit premier niveau d'excrétion de bicarbonate ;
du bicarbonate ayant été administré audit sujet avant d'avoir obtenu ledit premier échantillonnage d'urine et avant d'avoir obtenu ledit second échantillonnage d'urine ; et
du bicarbonate ayant été administré audit sujet entre 0 et 180 min avant d'avoir obtenu un premier échantillon dudit premier échantillonnage d'urine et avant d'avoir obtenu un premier échantillon dudit second échantillonnage d'urine.

2. Procédé selon la revendication 1, ledit bicarbonate ayant été administré sous la forme d'une provocation orale.

3. Procédé selon l'une quelconque des revendications précédentes, le procédé est en outre décrit par
- ledit premier niveau d'excrétion de bicarbonate dans ledit premier échantillonnage d'urine qui est réglé à un premier niveau d'excrétion de référence avant de déterminer l'efficacité du composé ; et/ou
- ledit second niveau d'excrétion de bicarbonate dans ledit second échantillonnage d'urine qui est réglé à un second niveau d'excrétion de référence avant de déterminer l'efficacité du composé.

4. Procédé selon la revendication 3, ledit premier niveau d'excrétion de référence étant un niveau d'excrétion de bicarbonate déterminé dans un échantillon d'urine provenant dudit sujet avant d'obtenir ledit premier échantillonnage d'urine et avant l'administration de bicarbonate ; et ledit second niveau d'excrétion de référence étant un niveau d'excrétion de bicarbonate déterminé dans un échantillon d'urine provenant dudit sujet avant d'obtenir ledit second échantillonnage d'urine et avant l'administration de bicarbonate.

5. Procédé selon l'une quelconque des revendications précédentes, ladite période de temps prédéfinie étant supérieure ou égale à 2 semaines, telle que supérieure ou égale à 3 semaines, comme supérieure ou égale à 4 semaines.

6. Procédé selon l'une quelconque des revendications précédentes, ledit premier et/ou ledit second niveau d'excrétion de bicarbonate étant mesuré en tant qu'ASC₀₋ₜ.

7. Procédé selon l'une quelconque des revendications précédentes, ledit bicarbonate ayant été administré audit sujet environ 30 min avant d'obtenir un premier échantillon dudit premier échantillonnage d'urine et avant d'obtenir un premier échantillon dudit second échantillonnage d'urine, telle qu'environ 60 min, comme environ 90 min, telle qu'environ 120 min, comme environ 150 min, telle qu'environ 180 min.

8. Procédé selon l'une quelconque des revendications précédentes, ledit bicarbonate ayant été administré en une quantité de 50-150 mg/kg, telle que 60-100 mg/kg, comme 70-90 mg/kg, telle que 75-80 mg/kg.

9. Procédé selon l'une quelconque des revendications précédentes, ledit premier échantillonnage d'urine et/ou ledit second échantillonnage d'urine étant un nombre d'échantillons d'urine obtenus individuellement.

10. Utilisation du niveau d'excrétion de bicarbonate dans l'urine pour déterminer l'efficacité d'un composé contre la mucoviscidose à l'aide d'un procédé tel que décrit dans l'une quelconque des revendications 1 à 9.
